(19) Europäisches Patentamt
European Patent Office
Office européen des brevets

(11) Veröffentlichungsnummer: **0 395 580 A2**

# (12) EUROPÄISCHE PATENTANMELDUNG

(21) Anmeldenummer: **90810305.4**

(22) Anmeldetag: **18.04.90**

(51) Int. Cl.[5]: **C07D 498/18, //(C07D498/18, 323:00,307:00,263:00)**

(30) Priorität: **26.04.89 CH 1595/89**

(43) Veröffentlichungstag der Anmeldung: **31.10.90 Patentblatt 90/44**

(84) Benannte Vertragsstaaten: **AT BE CH DE DK ES FR GB GR IT LI LU NL SE**

(71) Anmelder: **CIBA-GEIGY AG**
**Klybeckstrasse 141**
**CH-4002 Basel(CH)**

(72) Erfinder: **Kump, Wilhelm, Dr.**
**Friedrich-Oser-Strasse 10**
**CH-4105 Biel-Benken(CH)**

## (54) Verfahren zur Herstellung polycyclischer Verbindungen.

(57) Die Erfindung betrifft ein Verfahren zur Herstellung von Verbindungen der Formel

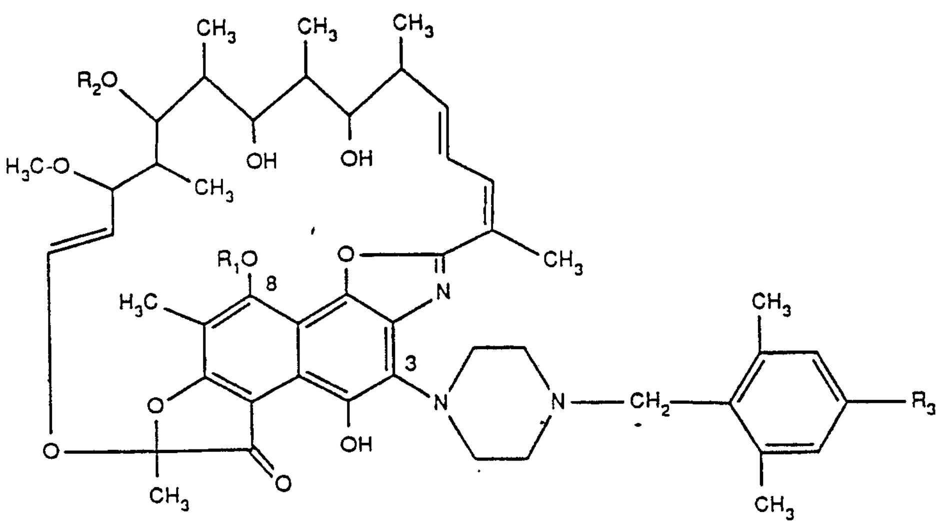

(I)

und ihrer Salze, worin $R_1$ Wasserstoff oder Trialkylacetyl bedeutet, $R_2$ Wasserstoff oder Acetyl bedeutet und $R_3$ Alkyl bedeutet, dadurch gekennzeichnet, dass man eine Verbindung der Formel

EP 0 395 580 A2

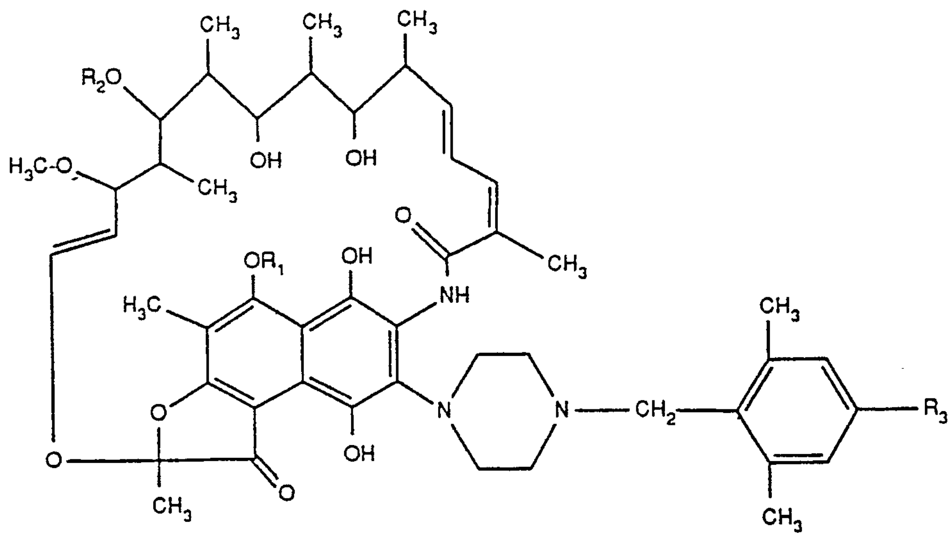

(II),

worin $R_1$ Trialkylacetyl ist, cyclisiert und gewünschtenfalls eine verfahrensgemäss oder auf andere Weise erhältliche Verbindung der Formel I oder ein Salz davon in eine andere Verbindung der Formel I oder ein Salz davon überführt, eine verfahrensgemäss erhältliche freie Verbindung der Formel I in ein Salz und/oder ein verfahrensgemäss erhältliches Salz in die freie Verbindung der Formel I oder in ein anderes Salz überführt.

## Verfahren zur Herstellung polycyclischer Verbindungen

Gegenstand der Erfindung ist ein neues Herstellungsverfahren für polycyclische Verbindungen der Formel

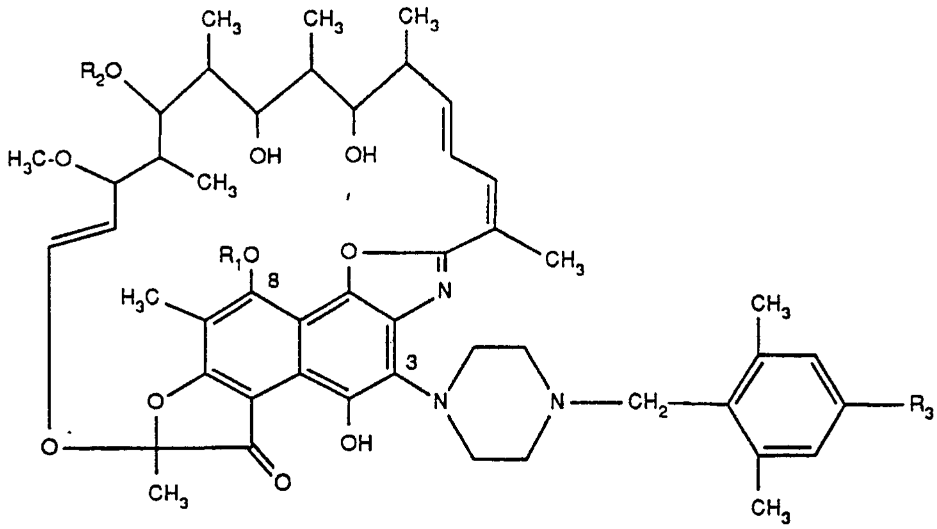

(I)

und ihrer Salze, worin $R_1$ Wasserstoff oder Trialkylacetyl bedeutet, $R_2$ Wasserstoff oder Acetyl bedeutet und $R_3$ Alkyl bedeutet.

Die zugrundeliegende Numerierung des Ringsystems entspricht derjenigen, die z.B. im US Patent Nr. 4,005,077 angewandt wurde.

Die Verbindungen der Formel I und ihre Salze sind in der Europäischen Patentanmeldung mit der Veröffentlichungsnummer 314,624 beschrieben.

Die Verbindungen der Formel I besitzen mehrere Chiralitätszentren, dementsprechend umfasst die vorliegende Erfindung auch die entsprechenden optischen Isomeren, z.B. Diastereoisomeren.

Die Verbindungen der Formel I können als, insbesondere pharmazeutisch verwendbare, Salze vorliegen. Da die erfindungsgemäss erhältlichen Verbindungen basische Zentren aufweisen, können sie somit Säureadditionssalze bilden. Diese werden beispielsweise mit anorganischen Säuren, wie Mineralsäuren, z.B. Schwefelsäure, eine Phosphor- oder Halogenwasserstoffsäure, oder mit organischen Carbonsäuren, wie gegebenenfalls, z.B. durch Halogen, substituierte $C_1$-$C_4$-Alkancarbonsäuren, z.B. Essigsäure, wie gegebenenfalls ungesättigte Dicarbonsäuren, z.B. Oxal-, Malon-, Bernstein-, Malein-, Fumar-, Phthal- oder Terephthalsäure, wie Hydroxycarbonsäuren, z.B. Glykol-, Milch-, Aepfel-, Wein- oder Citronensäure, wie Aminosäuren, z.B. Asparagin- oder Glutaminsäure, oder mit organischen Sulfonsäuren, wie gegebenenfalls, z.B. durch Halogen, substituierte $C_1$-$C_4$-Alkan- oder Arylsulfonsäuren, z.B. Methan-, Brombenzol- oder Toluolsulfonsäure, gebildet. Entsprechende Säureadditionssalze können auch mit dem zusätzlich vorhandenen basischen Zentrum gebildet werden. Ferner können die erfindungsgemässen Verbindungen mit einer aciden phenolischen Hydroxygruppe Salze mit Basen bilden, z.B. Alkalimetall-, wie Natrium- oder Kaliumsalze. Weiterhin können entsprechende innere Salze gebildet werden.

Trialkylacetyl bedeutet insbesondere Tri-$C_1$-$C_7$-alkylacetyl, bevorzugt Tri-$C_1$-$C_4$-alkylacetyl, wobei Alkyl jeweils die nachstehend angegebene Bedeutung hat. In erster Linie kommt Pivaloyl in Betracht.

Alkyl bedeutet insbesondere $C_1$-$C_7$-Alkyl und ist z.B. Methyl, Ethyl, Propyl, Isopropyl, n-Butyl, Isobutyl, sek.-Butyl, tert.-Butyl und umfasst ferner entsprechende Pentyl-, Hexyl-und Heptylreste. Bevorzugt ist $C_1$-$C_4$-Alkyl, in erster Linie jedoch Methyl.

Von Derivaten, die sich beispielsweise vom Rifamycin SV ableiten, ist bekannt, dass sie ausgeprägte antibiotische Eigenschaften besitzen und beispielsweise zur Behandlung von Tuberkulose eingesetzt werden können. Es wurde festgestellt, dass die Verbindungen der Formel I und ihre pharmazeutisch verwendbaren Salze in den üblichen pharmakologischen Testmodellen zur Prüfung keine entsprechende antibiotische Aktivität zeigen.

Ueberraschenderweise dagegen besitzen sie jedoch eine signifikante lipidsenkende Wirkung, die in Tierversuchen, vorzugsweise an Säugetieren, z.B. an Ratten, nachgewiesen werden kann (vergleiche EP-A-314,624).

Insbesondere dank ihrer LDL-senkenden Wirkung können die erfindungsgemässen Verbindungen z.B. als Hypolipidämika zur Behandlung von Hyperlipidämien, hauptsächlich der Typen IIa und IIb, und Atherosclerose, z.B. bei Vorliegen von Hyperlipoproteinämie als Risikofaktor, verwendet werden.

Dementsprechend können die Verbindungen der Formel I und ihre pharmazeutisch verwendbaren Salze z.B. als Pharmazeutika, beispielsweise als Hypolipidämika zur Behandlung von Hyperlipidämien, hauptsächlich der Typen IIa und IIb, und von Arteriosklerose bei Vorliegen von Hyperlipoproteinamie als Risikofaktor, verwendet werden.

Die Erfindung betrifft insbesondere die Herstellung von Verbindungen der Formel I und ihre Salze, worin $R_1$ Pivaloyl und $R_3$ Methyl bedeuten.

Die Erfindung betrifft insbesondere die in den Beispielen beschriebene Herstellungsweise.

Die Herstellung von Verbindungen der Formel I und ihrer Salze ist dadurch gekennzeichnet, dass man eine Verbindung der Formel

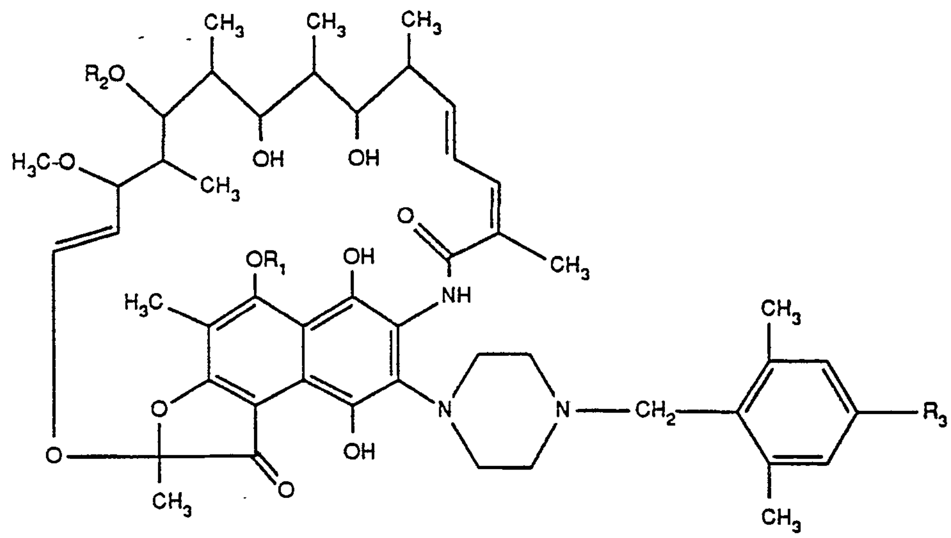

(II)

worin $R_1$ Trialkylacetyl ist, cyclisiert und gewünschtenfalls eine verfahrensgemäss oder auf andere Weise erhältliche Verbindung der Formel I oder ein Salz davon in eine andere Verbindung der Formel I oder ein Salz davon überführt, eine verfährensgemäss erhältliche freie Verbindung der Formel I in ein Salz und/oder ein verfährensgemäss erhältliches Salz in die freie Verbindung der Formel I oder in ein anderes Salz überführt.

Salze der Ausgangsmaterialien der Formel II, die eine acide phenolische Hydroxygruppe aufweisen, sind entsprechende Salze mit Basen der vorstehend aufgeführten Art, während entsprechende Ausgangsverbindungen mit den basischen Zentren auch entsprechende Säureadditionssalze analog der Säureadditionssalze der Formel I bilden können.

Die vor- und nachstehend beschriebenen Umsetzungen werden in an sich bekannter Weise durchgeführt, z.B. in Ab- oder üblicher Weise in Anwesenheit eines geeigneten Lösungs-oder Verdünnungsmittels oder eines Gemisches derselben, wobei man, falls erforderlich, in einem geschlossenen Gefäss, unter Druck, in einer Inertgasatmosphäre und/oder unter wasserfreien Bedingungen arbeitet.

Die Aufarbeitung des Reaktionsproduktes und Isolierung aus dem verfahrensgemäss erhältlichen Reaktionsgemisch erfolgt in an sich bekannter Weise, z.B. durch Verdünnen mit Wasser, und/oder gegebenenfalls durch Neutralisieren oder leichtes Ansäuern (bis etwa pH = 3) mit einer wässrigen Säure, wie einer anorganischen oder organischen Säure, z.B. einer Mineralsäure oder, vorteilhafterweise, Zitronensäure, und Zugabe eines mit Wasser nicht-mischbaren Lösungsmittels, wie eines chlorierten Kohlenwasserstoffs, z.B. Chloroform oder Methylenchlorid, wobei das Reaktionsprodukt in die organische Phase übergeht, aus welcher es in üblicher Weise, z.B. durch Trocknen, Eindampfen des Lösungsmittels und Kristallisation und/oder Chromatographie des Rückstandes oder andere übliche Reinigungsmethoden in gereinigter Form erhalten werden kann.

$R_1$ steht in erster Linie für Pivaloyl.

Die Cyclisierung von Verbindungen der Formel II erfolgt vorteilhaft unter Erwärmen, z.B. in einem Temperaturbereich von etwa 50° C bis zur Siedetemperatur des Reaktionssystems, z.B. bis etwa 180° C,

insbesondere in einem Temperaturintervall von etwa 100 °C bis etwa 170 °C.

Vorzugsweise erhält man solche Verbindungen der Formel I, worin $R_1$ Wasserstoff ist, und acyliert diese durch Behandeln mit einem der nachstehend aufgeführten Acylierungsmittel, insbesondere einem Pivaloylhalogenid, z.B. -chlorid.

Das Ausgangsmaterial der Formel II kann man beispielsweise herstellen, indem man Rifamycin S oder 3-Halogen-, insbesondere 3-Bromrifamycin-S mit einem Amin der Formel

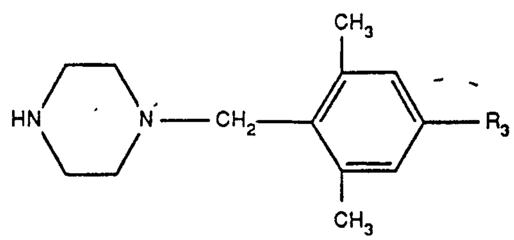

(IIa)

umsetzt. Dabei arbeitet man insbesondere mit einem Ueberschuss an Amin der Formel IIa beispielsweise in einem Temperaturbereich von etwa 0° bis etwa 100 °C. Es bildet sich ein Gemisch aus der Chinon- und der Hydrochinonform. Dieses Gemisch kann mittels Reduktion, z.B. mit katalytischer Hydrierung, in das entsprechende Hydrochinon (Derivat von Rifamycin-SV) übergeführt werden ($R_1$ = H). Durch Behandeln mit entsprechenden Acylierungsmitteln, z.B. mit einem Säureanhydrid, wie Pivaloylchlorid, in Gegenwart einer Base, wie Pyridin, kann man zu Verbindungen der Formel II gelangen, worin $R_1$ Trialkylacetyl bedeutet.

Die Erfindung betrifft ebenfalls die nach der vorstehenden Verfahrensvariante erhältlichen neuen Verbindungen.

Eine erfindungsgemäss oder auf andere Weise erhältliche Verbindung der Formel I oder Salz davon kann in an sich bekannter Weise in eine andere Verbindung der Formel I übergeführt werden.

Verbindungen der Formel I, worin $R_1$ Wasserstoff ist, können in an sich bekannter Weise acyliert werden, beispielsweise durch Umsetzung mit der entsprechenden Carbonsäure oder einem reaktionsfähigen Derivat davon. Derartige reaktionsfähige Derivate sind beispielsweise Anhydride, inklusive gemischte Anhydride, wie ein Säurehalogenid, z.B. -chlorid, oder Anhydride mit einem Ameisensäureester, aktivierte Carbonsäureester, wie Cyanmethyl-, (4-)Nitrophenyl-, Polyhalogenphenyl-, z.B. Pentachlorphenyl-, -ester. Die Umsetzung mit der Carbonsäure oder einem Salz davon erfolgt unter wasserabspaltenden Bedingungen, z.B. unter azeotroper Entfernung des Reaktionswassers, oder durch Behandeln mit einem geeigneten Kondensationsmittel, z.B. N,N′-Dicyclohexyl-carbodiimid. Die Umsetzung mit einem reaktionsfähigen Säurederivat wird vorteilhaft in Gegenwart einer Base durchgeführt. Entsprechend kann durch Behandeln mit einem entsprechenden Acetylierungsmittel der Acetylrest $R_2$ in Verbindungen der Formel I, worin $R_2$ Wasserstoff ist, eingeführt werden, gegebenenfalls nach reversiblem Schutz der OH-Gruppen an C-21 und C-23.

Durch Behandeln mit starken Basen, wie Alkalimetallhydroxiden, können der Acetylrest $R_2$ und der Acylrest $R_1$ durch Wasserstoff ersetzt werden. Der Acylrest $R_1$ kann auch in Gegenwart des Acetylrests $R_2$ selektiv abgespalten werden, beispielsweise durch Behandeln mit einem Fluorid, wie Alkalimetall-, z.B. Natrium- oder Cäsiumfluorid, oder einem Ammoniumfluorid, z.B. Tetrabutylammoniumfluorid.

Salze von Verbindungen der Formel (I) können in an sich bekannter Weise hergestellt werden. So erhält man beispielsweise Säureadditionssalze von Verbindungen der Formel (I) durch Behandeln mit einer Säure oder einem geeigneten Ionenaustauscherreagenz. Salze können in üblicher Weise in die freien Verbindungen überführt werden, Säureadditionssalze z.B. durch Behandeln mit einem geeigneten basischen Mittel.

Je nach Verfahrensweise bzw. Reaktionsbedingungen können die erfindungsgemässen Verbindungen mit salzbildenden, insbesondere basischen Eigenschaften, in freier Form oder bevorzugt in Form von Salzen erhalten werden.

Infolge der engen Beziehung zwischen der neuen Verbindung in freier Form und in Form ihrer Salze sind im Vorausgegangenen und nachfolgend unter der freien Verbindung sinn-und zweckgemäss gegebenenfalls auch die entsprechenden Salze bzw. die freie Verbindung zu verstehen.

Die neuen Verbindungen einschliesslich ihrer Salze von salzbildenden Verbindungen können auch in Form ihrer Hydrate erhalten werden oder andere zur Kristallisation verwendete Lösungsmittel einschliessen.

Die neuen Verbindungen können, je nach der Wahl der Ausgangsstoffe und Arbeitsweisen, in Form

eines der möglichen Isomeren oder als Gemische derselben, z.B. je nach der Anzahl der asymmetrischen Kohlenstoffatome, als reine optische Isomere, wie Antipoden, oder als Isomerengemische, wie Racemate, Diastereoisomerengemische oder Racematgemische, vorliegen.

Erhaltene Racematgemische können auf Grund der physikalisch-chemischen Unterschiede der Bestandteile in bekannter Weise in die reinen Isomeren oder Racemate getrennt aufgetrennt werden, beispielsweise durch fraktionierte Kristallisation.

Erhaltene Racemate lassen sich ferner nach bekannten Methoden in die optischen Antipoden zerlegen, beispielsweise durch Umkristallisation aus einem optisch aktiven Lösungsmittel, Chromatographie an chiralen Adsorbentien, mit Hilfe von geeigneten Mikroorganismen, durch Spaltung mit spezifischen, immobilisierten Enzymen, über die Bildung von Einschlussverbindungen, z.B. unter Verwendung chiraler Kronenether, wobei nur ein Enantiomeres komplexiert wird, oder durch Ueberführung in diastereomere Salze, z.B. durch Umsetzung eines basischen Endstoffracemats mit einer optisch aktiven Säure, wie Carbonsäure, z.B. Wein- oder Aepfelsäure, oder Sulfonsäure, z.B. Camphersulfonsäure, und Trennung des auf diese Weise erhaltenen Diastereomerengemisches, z.B. auf Grund ihrer verschiedenen Löslichkeiten, in die Diastereomeren, aus denen das gewünschte Enantiomere durch Einwirkung geeigneter Mittel freigesetzt werden kann. Vorteilhaft isoliert man das wirksamere Enantiomere.

Die Erfindung betrifft auch diejenigen Ausführungsformen des Verfahrens, nach denen man von einer auf irgendeiner Stufe des Verfahrens als Zwischenprodukt erhältlichen Verbindung ausgeht und die fehlenden Schritte durchführt oder einen Ausgangsstoff in Form eines Derivates bzw. Salzes und/oder seiner Racemate bzw. Antipoden verwendet oder insbesondere unter den Reaktionsbedingungen bildet.

Beim Verfahren der vorliegenden Erfindung werden vorzugsweise solche Ausgangsstoffe verwendet, welche zu den eingangs als besonders wertvoll geschilderten Verbindungen führen. Die neuen Ausgangsstoffe, die speziell für die Herstellung der erfindungsgemässen Verbindungen entwickelt wurden, insbesondere neue Verbindungen der Formel II, ihre Verwendung und Verfahren zu ihrer Herstellung bilden ebenfalls einen Gegenstand der Erfindung, wobei die Variablen $R_1$, $R_2$ und $R_3$ die für die jeweils bevorzugten Verbindungsgruppen der Formel I davon angegebenen Bedeutungen haben.

Das nachfolgende Beispiel illustriert die vorstehend beschriebene Erfindung; es soll diese jedoch in ihrem Umfang nicht einschränken. Temperaturen sind in Celsiusgraden angegeben.

Beispiel 1:

Eine 10%ige Lösung von frisch hergestelltem 8-O-Pivaloyl-3-[4-(2,4,6-trimethylbenzyl)-1-piperazinyl]-rifamycin-SV in Toluol wird im Druckgefäss während 15 Minuten auf 170° C erhitzt. Dann wird das Toluol verdampft. Das zurückbleibende Material kristallisiert aus Methanol/Wasser. Die nach zweimaliger Kristallisation erhal tenen Kristalle vom Smp. 175° stellen das 1-Desoxy-15-desoxo-1,15-oxy-3-[4-(2,4,6-trimethylbenzyl)-1-piperazinyl]-rifamycin der Formel I dar, worin $R_1$ Wasserstoff bedeutet, $R_2$ Acetyl bedeutet und $R_3$ Methyl bedeutet.

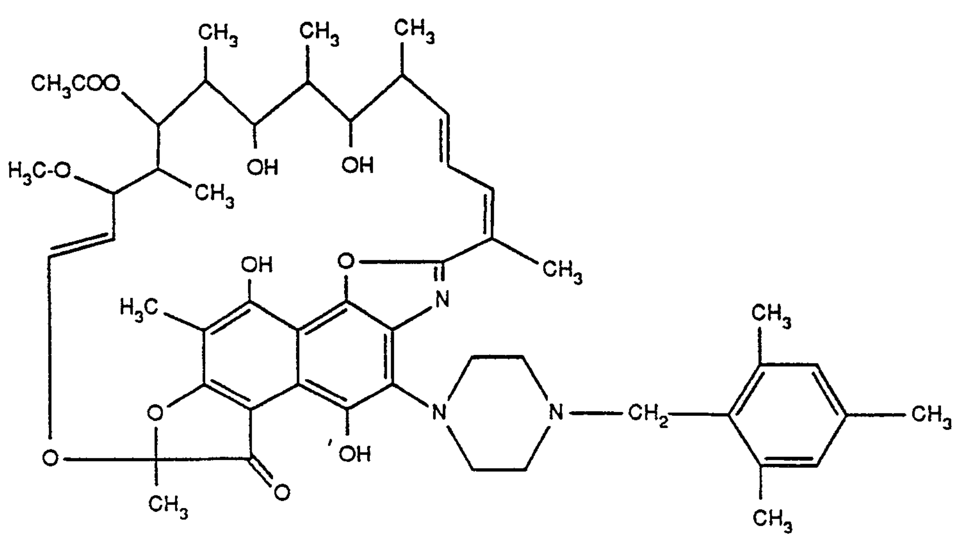

Das Ausgangsmaterial kann beispielsweise folgendermassen hergestellt werden:

a) Eine Lösung von 10 g 3-[4-(2,4,6-Trimethylbenzyl)-1-piperazinyl]-rifamycin-S in 100 ml Pyridin wird unter Rühren tropfenweise mit 1,5 g Pivalinsäurechlorid (1,13 Aequiv.) versetzt und bei 20° während 10 Minuten reagieren gelassen. Anschliessend setzt man dem Reaktionsgemisch 10 ml Methanol zu und rührt noch 1 Stunde weiter. Dann wird im Vakuum zur Trockne eingedampft, der Rückstand in Essigester gelöst, die Essigesterlösung mit wässriger Natriumbicarbonatlösung und gesättigter Kochsalzlösung gewaschen, über $Na_2SO_4$ getrocknet und eingedampft. Es bleibt 8-0-Pivaloyl-3-[4-(2,4,6-trimethylbenzyl)-1-piperazinyl)-rifamycin-S zurück, das aus Aether blauschwarze Kristalle vom Smp. 191-93° (Z) bildet (Sintern bei etwa 162°, sowie wiederum bei etwa 175°).

b) Das erhaltene Chinon wird in Tetrahydrofuran gelöst und der Lösung unter gutem Rühren ein Ueberschuss Zinkstaub und tropfenweise solange 1 N Salzsäure zugesetzt, bis das Reaktionsgemisch eine gelbe Farbe angenommen hat. Nun filtriert man das Reaktionsgemisch, wäscht die Tetrahydrofuranlösung zweimal mit gesättigter Kochsalzlösung, trocknet sie mit Natriumsulfat und dampft im Vakuum rasch bei niedriger Temperatur ein. Der gelbe Rückstand besteht aus 8-0-Pivaloyl-3-[4-(2,4,6-trimethylbenzyl)-1-pipera zinyl]-rifamycin-SV, welches in dieser Form direkt für die Ringschlussreaktion eingesetzt werden kann. Das Material kristallisiert aus Aether in orangegelben Kristallen, die bei etwa 165° unter Zersetzung schmelzen.

Beispiel 2:

Eine Lösung von 10 g 1-Desoxy-15-desoxo-1,15-oxy-3-[4-(2,4,6-trimethylbenzyl)-1-piperazinyl]-rifamycin in 100 ml Pyridin wird unter Rühren tropfenweise mit 1,5 g Pivalinsäurechlorid versetzt und bei 20° während 10 Minuten reagieren gelassen. Anschliessend setzt man dem Reaktionsgemisch 10 ml Methanol zu und rührt noch 1 Stunde weiter. Dann wird im Vakuum zur Trockne eingedampft, der Rückstand in Essigester gelöst, die Essigesterlösung mit wässriger Natriumbicarbonatlösung und gesättigter Kochsalzlösung gewaschen, über $Na_2SO_4$ getrocknet und eingedampft. Man erhält so das 8-O-Pivaloyl-1-desoxy-15-desoxo-1,15-oxy-3-[4-(2,4,6-trimethylbenzyl)-piperazin-1-yl]-rifamycin der Formel I, worin $R_1$ Pivaloyl bedeutet, $R_2$ Acetyl ist und $R_3$ Wasserstoff ist vom Smp. 160-165°.

**Ansprüche**

1. Verfahren zur Herstellung von Verbindungen der Formel

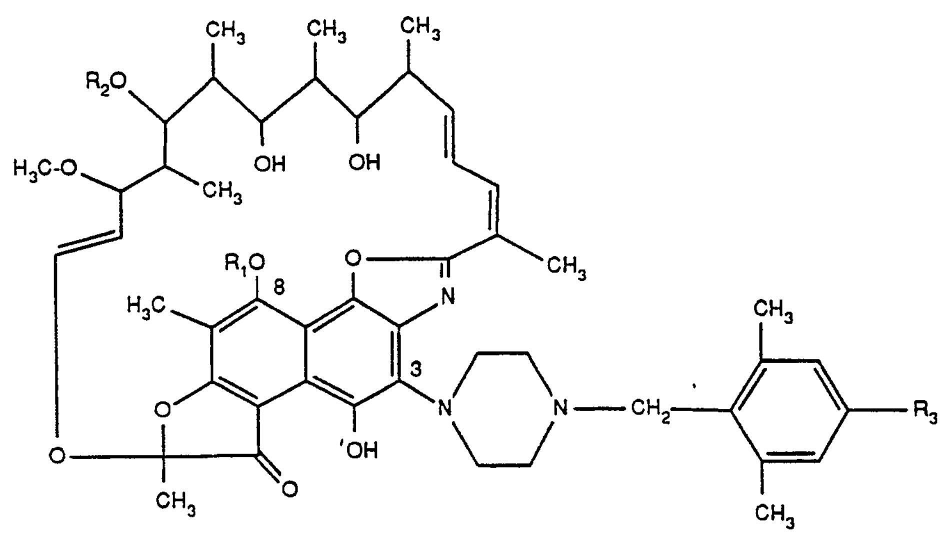

(I)

und ihrer Salze, worin $R_1$ Wasserstoff oder Trialkylacetyl bedeutet, $R_2$ Wasserstoff oder Acetyl bedeutet und $R_3$ Alkyl bedeutet, dadurch gekennzeichnet, dass man eine Verbindung der Formel

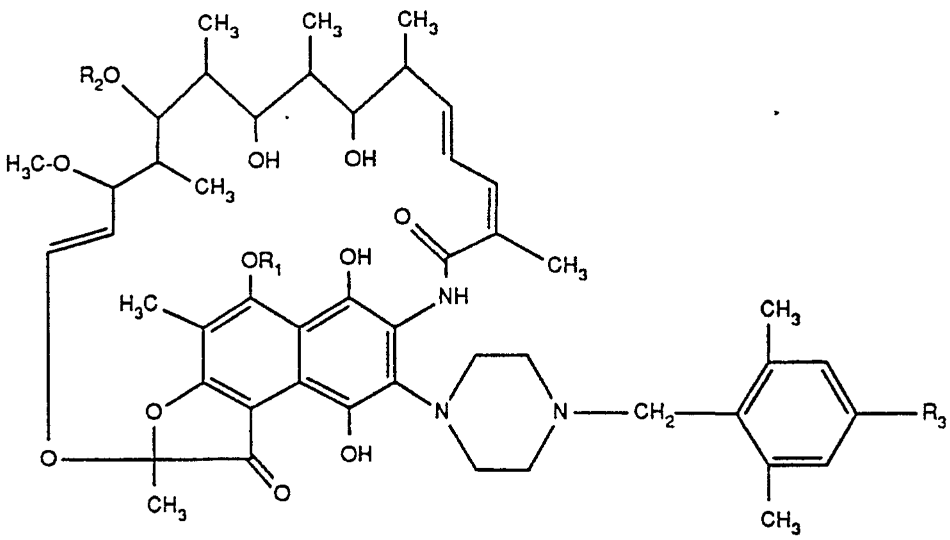

(II),

worin $R_1$ Trialkylacetyl ist, cyclisiert und gewünschtenfalls eine verfahrensgemäss oder auf andere Weise erhältliche Verbindung der Formel I oder ein Salz davon in eine andere Verbindung der Formel I oder ein Salz davon überführt, eine verfahrensgemäss erhältliche freie Verbindung der Formel I in ein Salz und/oder ein verfahrensgemäss erhältliches Salz in die freie Verbindung der Formel I oder in ein anderes Salz überführt.

2. Verfahren nach Anspruch 1, dadurch gekennzeichnet dass man die Cyclisierung in einem Temperaturbereich von etwa 50 °C bis zur Siedetemperatur des Reaktionssystems durchführt.

3. Verfahren nach Anspruch 3, dadurch gekennzeichnet dass man die Cyclisierung in einem Temperaturintervall von etwa 100 °C bis etwa 170 °C durchführt.

4. Verfahren nach einem der Ansprüche 1-3, dadurch gekennzeichnet, dass man, zur Herstellung einer Verbindung der Formel I, worin $R_1$ Trialkylacetyl bedeutet, eine verfahrensgemäss erhältliche Verbindung der Formel I, worin $R_1$ Wasserstoff ist, mit einem $R_1$ entsprechenden Acylierungsmittel behandelt.

5. Verfahren nach Anspruch 4, dadurch gekennzeichnet, dass man eine verfahrensgemäss erhältliche Verbindung der Formel I, worin $R_1$ Wasserstoff ist, mit einem Pivaloylhalogenid, wie -chlorid, umsetzt.

6. Verfahren nach einem der Ansprüche 1-5 zur Herstellung von Verbindungen der Formel I und ihrer Salze, worin $R_1$ Tri-$C_1$-$C_7$-alkyl-acetyl bedeutet und $R_3$ $C_1$-$C_7$-Alkyl bedeutet.

7. Verfahren nach einem der Ansprüche 1-5 zur Herstellung von Verbindungen der Formel I und ihrer Salze, worin $R_1$ Tri-$C_1$-$C_4$-alkyl-acetyl bedeutet und $R_3$ $C_1$-$C_4$-Alkyl bedeutet.

8. Verfahren nach einem der Ansprüche 1-5 zur Herstellung von Verbindungen der Formel I und ihrer Salze, worin $R_1$ Pivaloyl bedeutet und $R_3$ Methyl ist.

9. Verfahren nach einem der Ansprüche 1-3 zur Herstellung von 1-Desoxy-15-desoxo-1,15-oxy-3-[4-(2,4,6-trimethylbenzyl)-1-piperazinyl]-rifamycin oder eines Salzes davon.

10. Verfahren nach einem der Ansprüche 1-5 zur Herstellung von 8-O-Pivaloyl-1-desoxy-15-desoxo-1,15-oxy-3-[4-(2,4,6-trimethylbenzyl)-piperazin-1-yl]-rifamycin oder eines Salzes davon.

11. Die nach dem Verfahren gemäss einem der Ansprüche 1-10 erhältlichen Verbindungen.